# EUROPÄISCHE PATENTSCHRIFT

(11) **EP 3 167 787 B1**
(45) Veröffentlichungstag und Bekanntmachung des Hinweises auf die Patenterteilung: **27.02.2019**
(21) Anmeldenummer: 15194393.3
(22) Anmeldetag: 12.11.2015
(51) Int. Cl.: A47L 13/16, A47L 17/00, A61K 8/02, D06N 7/00

(54) **REINIGUNGSARTIKEL MIT EINEM ABSORBIERENDEN GRUNDMATERIAL**
CLEANING ITEM WITH AN ABSORBENT BASE MATERIAL
ARTICLE DE NETTOYAGE COMPRENANT UN MATERIAU DE BASE ABSORBANT

(43) Veröffentlichungstag der Anmeldung: 17.05.2017
(73) Patentinhaber: CMC Consumer Medical Care GmbH, 89567 Sontheim an der Brenz (DE)
(72) Erfinder: Mangold, Rainer, 89542 Herbrechtingen (DE); Römpp, Angela, 73105 Dürnau (DE); Arkhipova, Maria, 89231 Neu-Ulm (DE)
(74) Vertreter: DREISS Patentanwälte PartG mbB

(56) Entgegenhaltungen:
- US-A- 4 082 878
- US-A- 4 142 334
- US-A- 4 601 938
- US-A1- 2005 260 390

## Beschreibung

Die Erfindung betrifft einen Reinigungsartikel mit einem absorbierenden Grundmaterial, der durch ein Flachmaterial gebildet ist, mit einer ersten und einer zweiten Seitenfläche, wobei auf mindestens der ersten Seitenfläche eine Beschichtung vorgesehen ist, die der ersten Seitenfläche des Reinigungsartikels eine gegenüber der unbeschichteten ersten Seitenfläche erhöhte Reinigungskraft verleiht. Im Stand der Technik sind eine Vielzahl von Reinigungsartikel, so zur Flächenreinigung, insbesondere zur Haushaltsreinigung, als auch zur Hautreinigung, insbesondere zur Gesichtsreinigung bekannt.

So beschreibt beispielsweise die DE 20 2005 014 927 U1 eine Wattescheibe zum Reinigen und Peeling der Haut, wobei auf mindestens einer Außenseite des Reinigungsartikels ein Bindemittelauftrag in mindestens einem Teilbereich und mindestens ein weiterer von Bindemittelauftrag freier Teilbereich vorgesehen ist. Dabei kann der Bindemittelauftrag in verschiedenen Mustern, wie beispielsweise Wellenlinien, Gitternetzen, Punkten oder Schuppenmustern erfolgen. Bei den vorgesehenen Mustern handelt es sich um vergleichsweise kleinteilige Musterelemente.

Ebenso ist aus der WO 03/104544 A1 ein Reinigungswischtuch mit einem strukturierten Beschichtungsauftrag bekannt, wobei die Musterdichte des Beschichtungsauftrages vergleichsweise uniform ausgebildet ist.

Aus der US 4,082,878 ist ein absorbierendes Textilmaterial bekannt, das eine Beschichtung in Form eines kontinuierlichen sechseckigen Bienenwabenmusters mit einer Liniendicke von 1 mm und einem Abstand von etwa 10 mm aufweist.

Ein weiteres Reinigungstuch mit einer Beschichtung ist aus der US 4,601,938 A vorbekannt, das einen linienförmigen Musterauftrag aufweist und für den Einsatz in Krankenhäusern, Waschräumen und Küchen verwendet werden soll.

Ausgehend von diesem Stand der Technik ist es die Aufgabe der vorliegenden Erfindung, einen Reinigungsartikel bereitzustellen, der bei gleichzeitiger guter Reinigungswirkung ausreichend Absorptionsflächen für abzutragende Bestandteile bereitstellt und, sofern es sich um einen Reinigungsartikel zur Hautreinigung, insbesondere Gesichtsreinigung handelt, als angenehm zur Haut empfunden wird oder sofern es sich um einen Reinigungsartikel für Flächenreinigung, so insbesondere im Haushalt handelt, dennoch auch ausgleichende Bereiche, geeignet beispielsweise zum Polieren, Weichwischen, zur Verfügung stellt.

Die Erfindung löst diese Aufgabe durch einen Reinigungsartikel mit dem Merkmal des Anspruchs 1, bei dem die Beschichtung eine Vielzahl von Beschichtungslinien umfasst und wobei, wenn über die erste Seitenfläche des Reinigungsartikels ein imaginäres Gitternetz aus parallelen, äquidistanten, senkrecht zueinander verlaufenden Linien mit einer Kantenlänge von 7 mm eines jeden Gitterquadrats gelegt wird, die erste Seitenfläche im Bereich von mindestens 5 Gitterquadraten pro 25 cm² einen ersten Bedeckungsgrad an Beschichtung von mindestens 15% und im Bereich von mindestens 5 Gitterquadraten pro 25 cm² einen zweiten Bedeckungsgrad an Beschichtung von höchstens 8% aufweist.

Unter einem Reinigungsartikel im Sinne der Erfindung sollen neben kosmetischen Reinigungsartikeln, wie insbesondere Kosmetikpads, Kosmetiktücher, auch Reinigungsartikel für Flächenreinigung, wie insbesondere Reinigungstücher beispielsweise für Haushaltszwecke, insbesondere zur Geschirrreinigung, aber auch zur Flächenreinigung vorzugsweise im Küchen- oder Sanitärbereich, sowie zur Scheiben- und Fensterreinigung etc. verstanden werden.

Dabei soll unter Gitternetz aus parallelen, äquidistanten, senkrecht zueinander verlaufenden Linien eine solche Anordnung an Linien verstanden werden, bei der eine Schar aus parallelen, äquidistanten Linien durch eine zweite Schar aus parallelen, äquidistanten Linien unter Bildung von rechten Winkeln geschnitten wird. Bei dem so entstehenden Gitternetz, auf das der Reinigungsartikel beliebig auflegbar ist, d.h. es ist keine Vorzugsrichtung des Gitternetzes vorgesehen, sollen dann mindestens 5 Gitterquadrate pro 25 cm² einen ersten Bedeckungsgrad an Beschichtung von mindestens 15% und somit einen hohen Bedeckungsgrad aufweisen und mindestens 5 Gitterquadrate pro 25 cm² einen zweiten Bedeckungsgrad an Beschichtung von höchstens 8% aufweisen und somit einen vergleichsweise niedrigen Bedeckungsgrad. Dabei sind per Definition lediglich solche Gitterquadrate für die Ermittlung heranzuziehen, die vollständig im Bereich des Reinigungsartikels liegen. Gitterquadrate, die durch die Randlinie (Kante) des Reinigungsartikels geschnitten werden, bleiben für die Betrachtung der zu zählenden Gitterquadrate außer Betracht.

Die Angabe "pro 25 cm²" wird als Bezugsgröße verstanden. Bei der Auswertung wird die gesamte Seitenfläche des Reinigungsartikels betrachtet. Im Falle von Reinigungsartikeln mit einer von 25 cm² abweichenden geringeren oder größeren Dimension wird die auf der ersten Seitenfläche des Reinigungsartikels ermittelte Anzahl an Gitterquadraten mit erstem und zweitem Bedeckungsgrad entsprechend auf 25cm² normiert umgerechnet.

Dabei wird unter dem Bedeckungsgrad der durch die Beschichtung innerhalb eines Gitterquadrates abgedeckte Bereich bezogen auf die Fläche des Gitterquadrates verstanden. Für die Auswertung des Bedeckungsgrades der Beschichtung in Bezug auf die jeweiligen Gitterquadrate können beliebige Auswerteverfahren, z.B. Computerunterstützte Grafik- und Zeichenprogramme herangezogen werden.

Auf diese Weise kann ein Reinigungsartikel bereitgestellt werden, der aufgrund von Bereichen mit relativ hohem Bedeckungsgrad durch eine große Beschichtungsdichte, insbesondere einer großen Beschichtungsdichte an Beschichtungslinien, eine gute Peeling- bzw. Reinigungswirkung für Verschmutzungen besitzt. Auf der anderen Seite bietet ein solcher Reinigungsartikel aber aufgrund einer ebenfalls großen Anzahl an Bereichen, die lediglich einen geringen Bedeckungsgrad besitzen, eine ausreichende Absorptions- oder Aufnahmefläche für die von der zu reinigenden Oberfläche freigesetzten Komponenten, wie beispielsweise Schmutzpartikel, Hautpartikel, Makeup oder Feuchtigkeit. Solche Absorptions- oder Aufnahmeflächen können dann auch gleichzeitig sanft zur Haut sein, sofern z.B. ein Kosmetiktuch oder Kosmetikpad realisiert sein soll oder weisen auch eine gewisse Weichheit gegenüber harten Oberflächen auf, wenn z.B. ein Haushaltstuch realisiert sein soll. Dies wird insbesondere auch dadurch erreicht, dass die Kantenlänge der Gitterquadrate mit 7 mm groß ist gegenüber den im Stand der Technik bekannten kleinteiligen Mustern, so dass jeweils große zusammenhängende Reinigungsbereiche und große zusammenhängende Absorptionsbereiche geschaffen werden.

Nach einem bevorzugten Ausführungsbeispiel kann vorgesehen sein, dass die erste Seitenfläche im Bereich von mindestens 8, insbesondere mindestens 10, weiter insbesondere mindestens 15, weiter insbesondere mindestens 20, weiter insbesondere höchstens 35, weiter insbesondere höchstens 30, weiter insbesondere höchstens 25 Gitterquadraten pro 25 cm² einen ersten Bedeckungsgrad von mindestens 15% aufweist.

Darüber hinaus kann es auch bevorzugt vorgesehen sein, dass bei der Ausgestaltung der Beschichtung mit mindestens 10, insbesondere mindestens 15 Gitterquadraten pro 25 cm² mit dem ersten Bedeckungsgrad von mindestens 15 %, davon mindestens 5 Gitterquadrate einen Bedeckungsgrad von mindestens 20% aufweisen.

Hierdurch können die genannten Vorteile noch besser realisiert werden.

Insbesondere weisen die Gitterquadrate mit dem ersten Bedeckungsgrad von mindestens 15% eine Obergrenze des Bedeckungsgrades von insbesondere höchstens 50%, weiter insbesondere höchstens 40%, weiter insbesondere höchstens 30%, weiter insbesondere höchstens 25% auf.

Insbesondere vorteilhaft weist die Beschichtung einen Bedeckungsquotienten, ermittelt aus der Anzahl der Gitterquadrate pro 25cm² des zweiten Bedeckungsgrades von höchstens 8% geteilt durch die Anzahl der Gitterquadrate pro 25cm² des ersten Bedeckungsgrades von mindestens 15%, von mindestens 0,1, insbesondere mindestens 0,2, weiter insbesondere mindestens 0,3, weiter insbesondere mindestens 0,4, weiter insbesondere höchstens 3,0, weiter insbesondere höchstens 2,5, weiter insbesondere höchstens 2,0, weiter insbesondere höchstens 1,8, weiter insbesondere höchstens 1,6 auf. Durch diese relative Anordnung an Bereichen mit verhältnismäßig niedrigem Bedeckungsgrad zu Bereichen mit verhältnismäßig hohem Bedeckungsgrad kann den unterschiedlichen Einsatzzwecken des Reinigungsartikels Rechnung getragen werden. Insbesondere sind Reinigungsartikel mit einer Beschichtung mit einem Bedeckungsquotienten von 0,1 - 1,0 für eine gewünschte stärkere Reinigungs- bzw. Peelingwirkung einsetzbar, hingegen können Reinigungsartikel mit einem Beschichtungsquotienten von 1,1 - 3,0 auf den Aspekt eines ausgewogeneren Reibungs-/Reinigungs-Verhalten zu Absorptionsflächen abgestellt werden.

Für die Bereitstellung eines Reinigungsartikels mit Bereichen für Reinigungs-/Reibungswirkung und gleichzeitig mit Bereichen für Absorptionsflächen kann die Beschichtung der ersten Seitenfläche des Reinigungsartikels insbesondere vorteilhaft mindestens einen Bereich, insbesondere mindestens 2 Bereiche, weiter insbesondere eine Vielzahl an Bereichen aufweisen, in denen mindestens 2, insbesondere mindestens 3, insbesondere mindestens 4, weiter insbesondere höchstens 30, weiter insbesondere höchstens 25, weiter insbesondere höchstens 20 Gitterquadrate des ersten Bedeckungsgrades von mindestens 15% direkt benachbart angeordnet sind und insbesondere mindestens einen Bereich, insbesondere mindestens 2 Bereiche, weiter insbesondere eine Vielzahl an Bereichen aufweisen, in denen mindestens 2, insbesondere mindestens 3, weiter insbesondere mindestens 4 Gitterquadrate des zweiten Bedeckungsgrades von höchstens 8% direkt benachbart angeordnet sind.

Unter direkt benachbart werden die Gitterquadrate verstanden, die über eine gemeinsame Kante oder einen gemeinsamen Eckpunkt verbunden sind.

Dabei umfasst die Beschichtung des Reinigungsartikels Beschichtungslinien. Eine vollflächige Beschichtung des Reinigungsartikels im Sinne eines kontinuierlichen, flächendeckenden ununterbrochenen Auftrags ist nicht vorgesehen. Neben der Beschichtung in Form von Beschichtungslinien können jedoch auch partielle flächenhafte Beschichtungen oder punktförmige Beschichtungen vorgesehen sein.

Solche von den linienhaften Beschichtungen abweichende Beschichtungen sollen jedoch erfindungsgemäß bei der Bestimmung des Bedeckungsgrades mit berücksichtigt werden. D. h., die jeweiligen Bedeckungsgrade ergeben sich durch die in den jeweiligen Gitterquadraten angeordneten Beschichtungen unabhängig von deren Gestaltung.

Ausschließlich flächige bzw. ausschließlich homogen verteilte punktförmige Beschichtungen sind jedoch nicht erfindungsgemäß.
Unter linienförmige Beschichtung wird insbesondere ein Element verstanden, bei dem eine Linienbreite von mindestens 0,2 mm vorgesehen ist und die Linie eine Länge aufweist, die mindestens das Fünffache der Linienbreite beträgt.

Die linienförmigen Beschichtungen können dabei grundsätzlich sowohl gerade Linien als auch gekrümmte Linien, sowie sich schneidende gerade oder gekrümmte Linien aufweisen. Die Linienführung kann grundsätzlich sowohl durchgehend als auch zumindest bereichsweise unterbrochen gestaltet sein, sofern die Linie als solche klar erkennbar bleibt. D. h., es sind auch gestrichelte, strichpunktierte oder gepunktete Beschichtungslinien im Sinne der vorliegenden Erfindung denkbar. Insbesondere dürfen die unterbrochenen Stellen nicht länger als das Zehnfache, insbesondere nicht länger als das Achtfache, insbesondere nicht länger als das Sechsfache, insbesondere nicht länger als das Vierfache der Linienbreite dieser unterbrochenen Stelle benachbarten Linie sein.

Darüber hinaus kann vorgesehen sein, dass die Beschichtung eine Vielzahl von Einzelmustern aufweist oder daraus gebildet ist. Die Einzelmuster weisen insbesondere Beschichtungslinien auf oder sind insbesondere aus Beschichtungslinien gebildet. Dabei sollen unter Einzelmustern solche Muster verstanden werden, die als offene oder geschlossene Muster ausgebildet sind. Offene Muster sind dabei solche Muster, bei denen der Linienanfang keinen Kontakt mit dem Linienende aufweist, und geschlossene Muster solche, bei denen Anfang und Ende einer Linie nicht mehr ermittelt werden können, da diese miteinander verbunden sind. Darüber hinaus sollen lediglich solche Muster Einzelmuster gemäß der Erfindung sein, die sich nicht auf einen einfachen Punkt reduzieren lassen. D. h., ein Einzelmuster muss mehr als ein Punkt sein, wobei Einzelmuster vorzugsweise solche sind, bei denen sich die Beschichtungslinie nicht ausschließlich als eine Gerade in nur eine Vektorrichtung erstreckt, sondern bei denen das Linienmuster Krümmungen und/oder Knicke aufweist.

Neben diesen Einzelmustern können jedoch auch weitere Beschichtungen, beispielsweise in Form von punktförmigen oder auch flächenförmigen Mustern, vorgesehen sein.

Dabei kann vorgesehen sein, dass die Einzelmuster voneinander diskret und so angeordnet sind, dass sie nicht durch eine oder mehrere kontinuierlich von einer ersten Kante der ersten Seitenfläche zu einer gegenüberliegenden zweiten Kante der ersten Seitenfläche verlaufende durchgehende, insbesondere regelmäßig verlaufende durchgehende Beschichtungslinie bildbar sind. Hierdurch wird erreicht, dass die Beschichtungslinien nicht lediglich in einer Vorzugsrichtung verlaufen.

Als Ränder des Reinigungsartikels werden alle Ränder oder Kanten des Reinigungsartikels sowie Begrenzungen desselben verstanden.

Voneinander diskrete Einzelmuster sind solche, die entweder voneinander vollständig separiert sind, oder auch Einzelmuster, die sich auch tangieren, schneiden und/oder überlappen können. Das Einzelmuster ist trotz Tangierens, Schneidens und/oder Überlappens dabei noch aus seiner durch die durch die Beschichtungslinie vorgegebene Richtung umschriebene flächenhafte Erstreckung als Einzelmuster erkennbar. Unter Einzelmuster werden auch Mustergruppen verstanden, die insbesondere aus mindestens zwei gleichen und/oder verschiedenen Musterelementen zusammengesetzt sind. Dabei werden insbesondere Anordnungen als Mustergruppen verstanden, bei denen mindestens zwei Musterelemente nebeneinander und in Kontakt stehend angeordnet sind, und/oder insbesondere auch Mustergruppen, bei denen ein erstes Musterelement ein zweites oder weiteres Musterelement zumindest teilweise, insbesondere vollständig umgibt oder umläuft, wie beispielsweise konzentrische Anordnungen, insbesondere an Kreisen, Ovalen oder Dreiecken oder sonstigen Polygonen, oder ineinander liegende geometrische Figuren jeglicher Art, die sich in einem Punkt oder einem Linienabschnitt berühren. Die Beschichtung kann auch Kombinationen derartiger Mustergruppen aufweisen.

Es versteht sich, dass die, insbesondere aus Beschichtungslinien, gebildeten Einzelmuster von einem unbeschichteten Bereich zumindest teilweise, vorzugsweise vollständig, umgeben sind und/oder auch einen unbeschichteten Bereich umfassen, und diesen unbeschichteten Bereich zumindest teilweise, bevorzugt vollständig, umlaufen.

Nach einer besonders bevorzugten Ausführungsform kann vorgesehen sein, dass mindestens ein Einzelmuster auf mindestens der ersten Seitenfläche so ausgebildet ist, dass für jede in der Fläche des Reinigungsartikels verlaufende Richtung bzw. bei einem gekrümmten Reinigungsartikel für jede Tangentialrichtung an den Reinigungsartikel ein Abschnitt dieser Beschichtung senkrecht hierzu verläuft. Das bedeutet, dass für jede mögliche Richtung in dem Reinigungsartikel, sofern der Reinigungsartikel flach liegt, ein Abschnitt bzw. Bereich in der Beschichtung existiert, der senkrecht zu einer beliebigen Richtung in der Ebene verläuft. Durch die Gestaltung der linienförmigen Beschichtung mit einer Krümmung kann eine bessere Verteilung der Kräfte in verschiedene Richtungen erreicht werden. Die Peeling- und Abriebwirkung für Verschmutzungen kann insoweit verbessert werden. Nach einer weiter besonders bevorzugten Ausführungsform kann der Abschnitt punktförmig sein, wobei dann eine in diesem Punkt angelegte imaginäre Tangente immer senkrecht zu einer beliebigen Richtung in dem Reinigungsartikel verläuft. Besonders bevorzugt weisen mindestens 20%, insbesondere mindestens 40%, insbesondere mindestens 50%, insbesondere mindestens 60%, insbesondere mindestens 80%, insbesondere 100% der Einzelmuster mindestens einen Abschnitt auf, der zu einer beliebigen Richtung der Fläche des Reinigungsartikels senkrecht verläuft. Insbesondere ist bei mindestens 20%, insbesondere mindestens 40%, insbesondere mindestens 50%, insbesondere mindestens 60%, insbesondere mindestens 80%, insbesondere 100% der Einzelmuster dieser mindestens eine Abschnitt punktförmig ausgebildet und eine hieran angelegte imaginäre Tangente verläuft senkrecht zu einer beliebigen Richtung in der Fläche des Reinigungsartikels.

Die auf der mindestens ersten Seitenfläche des Reinigungsartikels vorgesehenen linienförmigen Beschichtungen können gleiche oder verschiedene geometrische Formen aufweisen und insbesondere in gleichen oder verschiedenen Abmessungen/Dimensionen ausgestaltet sein. Insbesondere können auch linienförmige Muster mit flächenförmigen oder punktförmigen Mustern kombiniert sein.

Darüber hinaus kann bevorzugt wenigstens ein Einzelmuster als Mustergruppe gebildet sein, die mindestens zwei aus Beschichtungslinien gebildete Musterelemente umfasst. Besonders bevorzugt sind mindestens 20%, insbesondere mindestens 40%, insbesondere mindestens 50%, insbesondere mindestens 60%, insbesondere mindestens 80% der Einzelmuster aus einer Mustergruppe gebildet. Weiter insbesondere ist jedes Einzelmuster aus einer Mehrzahl von Musterelementen aufgebaut. Die Mustergruppe kann beispielsweise aus inneren und äußeren Musterelementen aufgebaut sein und/oder sich zu einem Gesamtmuster zusammenfügenden Musterelementen oder weiteren Musterelementen, die z. B. nebeneinander und sich dabei berührend angeordnet sind, aufgebaut sein. Besonders bevorzugt kann die Ausgestaltung einer Mustergruppe vorgesehen sein, derart dass ein Musterelement eines Einzelmusters ein zweites Musterelement oder weitere Musterelemente zumindest bereichsweise, insbesondere jedoch vollständig umläuft. Dabei soll unter Umlaufen auch verstanden werden, dass die Linien mindestens bereichsweise einander berühren oder miteinander gleichlaufend ausgebildet sind. Insbesondere kann die Mustergruppe eines Einzelmusters so ausgestaltet sein, dass ein erstes Musterelement einem zweiten Musterelement oder weiteren Musterelementen nebeneinander zugeordnet und sich dabei berührend angeordnet ist. Es sind auch Kombinationen an Mustergruppen denkbar. Insgesamt kann durch Mustergruppen die abrasive Wirkung weiter verbessert werden.

Dabei kann insbesondere vorgesehen sein, dass die Einzelmuster von einem unbeschichteten äußeren Bereich umgeben sind, der eine von der geometrischen Form der Einzelmuster verschiedene geometrische Form aufweist.

In diesem Fall wird erreicht, dass anders als bei Gitter- oder Streifenmustern keine Vorzugsrichtung besteht, sondern eine Fläche für Absorption, Drapierbarkeit allseitig gleich gut bereitgestellt werden kann. Ähnliche Effekte können insbesondere auch dadurch erzielt werden, dass verschiedene Beschichtungen, z. B. linienförmige Beschichtungen, mit anderen wie punktförmigen oder flächenförmigen Beschichtungen kombiniert werden.

Besonders bevorzugt ist, dass mindestens ein Einzelmuster auf der mindestens ersten Seitenfläche allseitig durch einen unbeschichteten äußeren Bereich umschlossen ist. Besonders bevorzugt ist weiter, dass eine Vielzahl von Einzelmustern je Reinigungsartikel, die auf der ersten Seitenfläche aufgebracht sind, allseitig von einem unbeschichteten äußeren Bereich umgeben ist. Insbesondere bevorzugt sind alle Einzelmuster des Reinigungsartikels von einem unbeschichteten äußeren Bereich umgeben. Auf diese Weise kann erreicht werden, dass einerseits durch die insbesondere linienförmigen Beschichtungen der Einzelmuster die Reinigungs- und Reibungswirkung des Reinigungsartikels in den jeweiligen Bereichen erhöht werden kann. Auf der anderen Seite bleiben gleichzeitig die erwünschten Eigenschaften des Grundmaterials, wie beispielsweise Absorptionseigenschaften, sowie taktile Eigenschaften des Reinigungsartikels erhalten.

Je nach gewünschtem Grad der abrasiven Eigenschaften sowie optischen und gestalterischen Wünschen kann vorgesehen sein, die Vielzahl an Beschichtungen in einem regelmäßigen Rapport aufzubringen oder nicht regelmäßig anzuordnen.

Dabei ist es insbesondere vorgesehen, dass die Beschichtung des Reinigungsartikels und hier insbesondere die erste Seitenfläche im Wesentlichen in ihrer gesamten Erstreckung umfasst, also nicht nur spezielle Bereiche, wie Mitte oder äußerer Bereich des Reinigungsartikels. Es ist daher bevorzugt vorgesehen, dass sich die Beschichtung über die gesamte mindestens erste Seitenfläche des Reinigungsartikels erstreckt, wobei je nach vorgesehenem Muster einzelne Bereiche des Reinigungsartikels, wie beispielsweise der Mittel- oder Randbereich, bezüglich der Musterdichte im Rahmen der vorgegebenen Erfindung variieren können. Es ist auch denkbar, in manchen Bereichen des Reinigungsartikels andere Muster anzubringen als in anderen Bereichen. So kann beispielsweise eine andere Beschichtung im Mittelbereich als im Randbereich vorgesehen sein. Weiterhin kann vorgesehen sein, dass die Beschichtung gleichmäßig oder ungleichmäßig über die Fläche des Reinigungsartikels verteilt vorgesehen sein kann.

Vorzugsweise ist nur die erste Seitenfläche beschichtet. Nach einer anderen Ausgestaltung können jedoch auch beide Seitenflächen beschichtet sein.

Besonders bevorzugt kann der Reinigungsartikel einen Bedeckungsgrad durch die Beschichtung von mindestens 6%, insbesondere mindestens 8%, weiter insbesondere mindestens 10%, weiter insbesondere mindestens 15%, weiter insbesondere mindestens 20% und insbesondere von höchstens 50%, weiter insbesondere höchstens 40%, weiter insbesondere höchstens 30% und weiter insbesondere höchstens 25% aufweisen. Auf diese Weise wird eine gute Reinigungswirkung des Reinigungsartikels erreicht und die erwünschten Eigenschaften des Grundmaterials, wie z.B. Absorptionsfähigkeiten, nicht zu stark geändert, sondern erhalten.

Sofern es sich bei der Beschichtung auf dem Reinigungsartikel um geschlossene Muster handelt, kann vorgesehen sein, dass die Einzelmuster in Summe einen Flächenanteil von mindestens 10%, insbesondere mindestens 20%, weiter insbesondere mindestens 30%, weiter insbesondere mindestens 40% und insbesondere höchstens 70%, weiter insbesondere höchstens 60%, weiter insbesondere höchstens 50% der mindestens ersten Seitenfläche einnehmen. Als Fläche eines Einzelmusters wird hierbei der durch die äußeren Beschichtungslinien (inklusive der Beschichtungslinien) eingeschlossene Bereich verstanden, somit sind auch die inneren unbeschichteten Bereiche des Einzelmusters oder bei Ausführung als Mustergruppe die dazugehörigen Flächen der einzelnen Musterelemente mit berücksichtigt. Sofern Einzelmuster geschlossene Musterbereiche aufweisen, von denen sich weitere, offene, also nicht in sich geschlossene Beschichtungslinien wegerstrecken, so bleiben diese freien anhängenden Beschichtungslinien bei der Berechnung der Fläche des Einzelmusters unberücksichtigt. Über die durch die Einzelmuster abgedeckten Flächen können ausreichende abrasive Eigenschaften unter Erhalt der dem Grundmaterial innewohnenden Eigenschaften wie Weichheit sowie Absorptionseigenschaften erreicht werden.

Ein Einzelmuster weist vorzugsweise eine Fläche mit einem Erstreckungsabstand zwischen den äußeren Beschichtungslinien von wenigstens 0,3 cm, vorzugsweise wenigstens 0,5 cm, weiter vorzugsweise wenigstens 0,7 cm, weiter vorzugsweise wenigstens 1,0 cm, weiter vorzugsweise wenigstens 1,5 cm, weiter vorzugsweise wenigstens 2 cm, weiter vorzugsweise höchstens 5 cm, weiter vorzugsweise höchstens 4 cm, weiter vorzugsweise höchstens 3 cm auf. Als Erstreckungsabstand, der z. B. ein Durchmesser sein kann, wird dabei die Distanz zwischen den jeweils distal am weitesten voneinander entfernt gelegenen Beschichtungslinien verstanden, die ein Einzelmuster beschreiben bzw. eingrenzen. Dabei wird an der äußeren Kante der Beschichtungslinie, also einschließlich deren Linienbreite gemessen.

Ein Einzelmuster weist, inklusive der umschreibenden Beschichtungslinien, vorzugsweise eine Fläche von mindestens 0,2 cm², weiter vorzugsweise von mindestens 0,5 cm², weiter vorzugsweise von mindestens 1,0 cm², weiter vorzugsweise von mindestens 1,5 cm², weiter vorzugsweise von höchstens 10,0 cm², weiter vorzugsweise von höchstens 8,0 cm², weiter vorzugsweise von höchstens 6,0 cm² auf.

Die Beschichtungsmerkmale können hinsichtlich ihrer geometrischen Form und/oder ihrer Abmessungen verschieden oder gleich ausgebildet sein. Hierbei können die verschiedenen Eigenschaften des Reinigungsartikels wie Bedeckungsgrad, Reibungskraft, Absorptionseigenschaften Berücksichtigung finden und durch die Einstellung der Beschichtungsmuster realisiert sein.

Besonders bevorzugt sind Beschichtungsmuster mit gekrümmten oder abgerundeten Bereichen, da diese im Gebrauchszustand eine bessere ergonomische Anpassung an die zu reinigende Oberfläche und eine schönere Anmutung ermöglichen.

Die Linienbreite der Beschichtungslinie kann mindestens 0,2 mm, insbesondere mindestens 0,4 mm, insbesondere mindestens 0,5 mm und weiter insbesondere mindestens 0,6 mm betragen. Dabei sollte die Linienbreite vorzugsweise höchstens 2,0 mm vorzugsweise, weiter insbesondere höchstens 1,6 mm, weiter insbesondere höchstens 1,2 mm, weiter insbesondere höchstens 1,0 mm betragen. Die Länge der Beschichtungslinie im Verhältnis zur Linienbreite soll insbesondere mindestens das 5-fache, vorzugsweise mindestens das 6-fache, weiter bevorzugt mindestens das 8-fache und weiter bevorzugt mindestens das 10-fache der Linienbreite ausmachen.

Die Höhe der Beschichtungslinien sowie gegebenenfalls vorgesehener weiterer Beschichtungen soll mindestens 0,1 mm, insbesondere mindestens 0,2 mm betragen. Dabei soll die Höhe der Beschichtungslinie insbesondere höchstens 0,8 mm, weiter insbesondere höchstens 0,6 mm und weiter insbesondere höchstens 0,4 mm sein. Die Messung der Höhe kann mit einem Mikroskop mit einer entsprechenden Vergrößerung ermittelt werden, und zwar dabei als die Differenz zwischen einer gemittelten oberen Oberseite des Grundmaterials und der oberen Kante der Beschichtungslinie. Vorzugsweise ist die gesamte Beschichtung über der oberen Oberseite des Grundmaterials erhaben ausgebildet.

Mit diesen bevorzugten Höhen der Beschichtungslinien und der gegebenenfalls vorgesehenen weiteren Beschichtungen werden vorteilhaft unangenehm anfühlbare haptische Effekte vermieden.

Das Flächengewicht der Beschichtung kann mindestens 5 g/m², insbesondere mindestens 10 g/m², weiter insbesondere mindestens 15 g/m² und weiter insbesondere mindestens 20 g/m² betragen. Nach oben hin soll das Flächengewicht vorzugsweise auf 50 g/m², weiter insbesondere auf höchstens 30 g/m² und weiter insbesondere auf höchstens 25 g/m² begrenzt sein.

Die Beschichtung ist insbesondere polymerbasiert. Insbesondere ist die Beschichtung auf Basis eines Polymers entnommen aus der Gruppe umfassend PE (Polyethylen), PP (Polypropylen), APAO (amorphe Polyalphaolefine), EVA (Ethylenvinylacetat), EVAC (Ethylenvinylacetat-Copolymere), PA (Polyamide), TPE-O (Thermoplastische Elastomere auf Olefinbasis), TPE-V (Vernetzte thermoplastische Elastomere auf Olefinbasis), TPE-E (Thermoplastische Copolyester), TPE-U (Thermoplastische Elastomere auf Urethanbasis), TPE-A (Thermoplastische Copolyamide, z.B. PEBA), TPE-S (Thermoplastische Styrol-Blockcopolymere), wie z.B. HSBC (hydrierte Styrol-Blockcopolymere), SEBS (Styrol-Ethylen-Butadien-Styrol-Polymere), SBS (Styrol-Butadien-Styrol), SEPS (Styrol-Ethylen-Propylen-Styrol) oder einer Kombination aus einer oder mehreren der genannten Polymere hiervon.

Vorzugsweise ist die Beschichtung in ihrer Zusammensetzung homogen. Vorzugsweise sind der Beschichtung keine abrasiv wirkenden Partikel beigefügt.

Als Materialien für die Beschichtung kommen bevorzugt Materialien mit einer Shore A-Härte von mindestens 30, insbesondere von mindestens 40, insbesondere von mindestens 50, weiter insbesondere mindestens 60 und insbesondere von höchstens 95, weiter insbesondere von höchstens 90, weiter insbesondere höchstens 80, weiter insbesondere höchstens 70 in Frage. Die Shore A-Härte stellt einen Werkstoffkennwert für Elastomere und Kunststoffe dar. Die Shore-A-Härte wird hierbei nach folgender Methode bestimmt.

### Methode zur Bestimmung der Shore-A Härte:

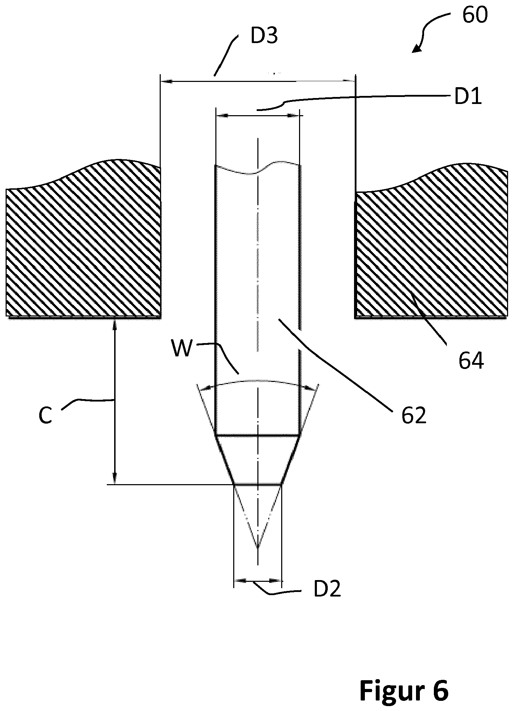

Die Shore-A-Härte ist ein Maß für den Widerstand eines Materials gegen das Eindringen eines Körpers von bestimmter Form und unter einer definierten Federkraft. Dabei gibt bei den Shore-Härte-Einheiten der Wert 0 die kleinste und der Wert 100 die größte Härte an.
Die Messung erfolgt in Anlehnung an die Normen DIN 53505:2000-08 und ISO 868:2003(E). Es wird dabei ein Härteprüfgerät nach Shore A eingesetzt. Ein solches Härteprüfgerät nach Shore A, das schematisch in Figur 6 mit dem Bezugszeichen 60 dargestellt ist, verwendet einen federbelasteten Eindruckkörper mit der Geometrie eines Kegelstumpfes. Der Eindruckkörper 62 aus Stahl hat einen Durchmesser D1 von 1,25 ± 0,15 mm, der in einen unteren Kegelstumpf mit einer unteren Fläche mit einem Durchmesser D2 von 0,79 ± 0,01 mm mit einem Neigungswinkel W von 35° ± 0,25° mündet. Der Abstand C zwischen der unteren Kante eines Druckfußes 64 und der unteren Fläche des Eindruckkörpers beträgt 2,5 ± 0,02 mm. Der Eindruckkörper ist innerhalb des Druckfußes 64 mit einer Ausnehmung mit einem Durchmesser D3 von 3 ± 0,5 mm zentriert eingebracht.

Die Prüfung soll an mechanisch nicht vorbeanspruchten Probekörpern durchgeführt werden. Für die Prüfung soll der Prüfkörper bereits 16 Stunden auspolymerisiert bzw. ausvulkanisiert sein. Die Prüfung wird bei Standardbedingungen bei 23 ± 2° C und 50 ± 2% Luftfeuchtigkeit durchgeführt. Die Probenkörper und die Geräte sind mindestens 1 Stunde lang entsprechend konditioniert.

Die Probenkörper brauchen Abmessungen, die Messungen wenigstens 12 mm von jeder Kante entfernt erlauben, und dabei über eine ausreichend planparallele Auflagefläche verfügen, damit der Druckfuß den Probenkörper auf einer Fläche im Radius von mindestens 6 mm um die Spitze des Eindruckkörpers berühren kann. Es sind Probenkörper mit einer Materialstärke von mindestens 4 mm erforderlich. Bei geringen Dicken können die Probenkörper aus mehreren dünneren Schichten zusammengesetzt sein. An jedem Probenkörper wird an mindestens 5 verschiedenen Stellen gemessen, wobei der Abstand von den Kanten des Probenkörpers mindestens 12 mm beträgt. Der Abstand zwischen den Messstellen soll mindestens 6 mm betragen. Das Andrückgewicht des Eindruckkörpers beträgt 1 kg.

Die Messzeit beträgt 3 Sekunden, d.h. die Härte wird 3 Sekunden nach der Berührung der Auflagefläche des Prüfgerätes und des Probekörpers abgelesen.

Das Aufbringen der Beschichtungslinien erfolgt dabei vorzugsweise mittels einer Walze, die eine dem Muster (Summe der Einzelmuster) entsprechende Gravur aufweist. Die erste Seitenfläche mit der Beschichtung kann einen dynamischen Reibungskoeffizienten gemessen in Anlehnung an ASTM D 1894-01 von mindestens 0,2, insbesondere mindestens 0,3, weiter insbesondere mindestens 0,4, weiter insbesondere mindestens 0,5, weiter insbesondere mindestens 0,6, weiter insbesondere mindestens 0,7, aufweisen, wobei Höchstwerte insbesondere von höchstens 1,5, insbesondere von höchstens 1,2, weiter insbesondere höchstens 1,0 erreicht werden sollen. Hierdurch werden ausreichende Reibungskräfte und Reinigungskräfte erzeugt.

### Test zur Ermittlung des dynamischen Gleitreibungskoeffizienten:

Vorliegend soll das Rutschverhalten von erfindungsgemäßen Reinigungsartikels mit Beschichtung ermittelt werden. Hierbei wird die mit der Beschichtung versehene erste Seitenfläche des Reinigungsartikels gegenüber einer standardisierten Oberfläche gezogen. Die hierbei auftretende Gleitreibungskraft A soll gemessen und hieraus dann der dynamische Gleitreibungskoeffizient ermittelt werden. Die Prüfmethode ist angelehnt an die ASTM D 1894-01, zur Bestimmung des Reibungsverhaltens von Kunststoff-Folien.

Die Prüfkörper müssen mindestens 2 Stunden im Normklima bei 23° C ± 2°C und 50% ± 2% Luftfeuchtigkeit konditioniert sein. Die Proben dürfen nicht geknickt, gefaltet oder zerkratzt sein; sonstige Veränderungen und Verunreinigungen sind zu vermeiden. Dasselbe gilt für die Prüfplatte aus Stahl. Das Prüfverfahren ist ebenso unter Normbedingungen (23°C ± 2°C, 50 % ± 2%) durchzuführen.

Aus dem Reinigungsartikel mit Beschichtung oder aus einer entsprechenden Rollenware wird ein Probenkörper einer Abmessung von 50 x 50 mm oder einem Durchmesser von 50 mm ausgestanzt und faltenfrei an einem Reibklotz befestigt. Bei der Rollenware handelt es sich aber um exakt dasjenige Material, aus dem die erfindungsmäßen Reinigungsartikel ausgestanzt oder ausgeschnitten werden.

Der Reibklotz weist eine Grundfläche von 63 mm x 63 mm Kantenlänge, also eine Kontaktgrundfläche von 40 cm² und eine Masse von 200 g ± 5g auf. Er wird über einen Faden (ohne Eigendehnung)am Kraftaufnehmer einer Zugprüfmaschine nach DIN 51 221 Klasse 1 befestigt. Eine solche Zugprüfmaschine ist das Prüfgerät Zwick Roell Typ Z010 von der Firma Zwick GmbH&Co.KG, 89079 Ulm, Deutschland.

Das Zusatzgerät bestehend aus dem Probentisch und Reibklotz nach DIN EN ISO 8295:2014 wird ebenfalls von der Firma Zwick angeboten. Der Reibklotz mit dem Probenkörper wird auf einen definierten Werkstoff, eine glatt polierte Stahlplatte (DIN EN 1939: 2003-12) vorsichtig aufgelegt. 15 Sekunden nach dem Auflegen des Reibklotzes wird der Versuch gestartet. Die Prüfgeschwindigkeit beträgt 150 mm/min, sowohl für den eigentlichen Messweg von 130 mm, als auch für den Vor- und Nachmessweg von jeweils 10 mm. Für die Ermittlung des dynamischen Gleitreibungskoeffizienten µ wird nur der Kraftverlauf des Messwegs von 130 mm herangezogen. Der Test wird für wenigstens fünf Prüfkörper durchgeführt. Es wird ein Mittelwert x und die Standardabweichung s auf zwei Nachkommastellen gerundet angegeben. Der dynamische Gleitreibungskoeffizient ergibt sich aus dem Quotienten der so ermittelten Gleitreibungskraft A ausgedrückt in Gramm (g) durch die durch den Reibklotz ausgeübte Kraft von 200 g.

Der Reinigungsartikel kann dabei bezüglich des Grundmaterials ein- oder mehrlagig ausgebildet sein und insbesondere ein Fasermaterial, insbesondere ein Vliesmaterial umfassen. Die Vliesmaterialien umfassen vorzugsweise natürliche cellulosebasierte Fasern, wie beispielsweise Baumwolle oder Viskose, oder synthetische Fasern, wie beispielsweise Polyester, vorzugsweise Polyester-Bikomponenten-Fasern oder Polyester-Mikrofaser, Polypropylen oder Mischungen daraus. Das Vliesmaterial kann insbesondere ein Vlies aus Endlosfasern oder vorzugsweise ein Vlies aus Stapelfasern sein.

Das Grundmaterial weist im Falle einer voluminösen Ausgestaltung des Reinigungsartikels, insbesondere sofern es sich um eher watteartige Produkte, wie insbesondere um Kosmetikpads handelt, insbesondere auch bei mehrlagigem Grundmaterial, eine Basislage mit einem Flächengewicht vorzugsweise von mindestens 80 g/m², weiter vorzugsweise von mindestens 100 g/m², weiter vorzugsweise von mindestens 120 g/m², weiter vorzugsweise von mindestens 150 g/m², weiter vorzugsweise von mindestens 200 g/m², weiter vorzugsweise von höchstens 300 g/m², weiter vorzugsweise von höchstens 280 g/m² auf. Bei einer eher tuchartigen Gestaltung des Reinigungsartikels kann das Flächengewicht vorzugsweise höchstens 100 g/m², bevorzugt mindestens 30 g/m² und besonders bevorzugt mindestens 40 g/m² betragen.

Vorzugsweise beträgt die Dicke des Reinigungsartikels, dabei einschließlich der Beschichtung auf der ersten Seitenfläche oder gegebenenfalls einer Beschichtung auf der zweiten Seitenfläche 0,3 - 5 mm, vorzugsweise 1 - 3 mm. Bei tuchartiger Gestaltung des Reinigungsartikels liegt die Dicke vorzugsweise bei 0,3 - 1,5 mm, bei einer voluminösen Ausgestaltung des Reinigungsartikels, insbesondere sofern es sich um eher watteartige Produkte, wie insbesondere um Kosmetikpads handelt, liegt die Dicke vorzugsweise bei 1,0 - 5 mm. Die Bestimmung der Dicke des Reinigungsartikels (inklusive der Beschichtung) wird unter Anwendung eines spezifischen Messdrucks von 0,5 kPa auf einer Tasterfläche von 25 cm² durchgeführt. Insbesondere kann ein Dickenmessgerät DMT der Firma Schröder verwendet werden. Im Übrigen wird die Dicke in Anlehnung an DIN EN ISO 9073-2: 1995 bestimmt.

Insbesondere bevorzugt weist der Reinigungsartikel nur auf der ersten Seitenfläche die erfindungsgemäße Beschichtung auf. Damit kann die zweite beschichtungsfreie Seitenfläche des Reinigungsartikels in weiteren, dem Reinigungs- und Peelingseffekt der ersten Seitenfläche vor- und/oder nachgeschalteten Benutzungshandlungen eingesetzt werden.

Insbesondere bevorzugt weist der Reinigungsartikel auf der ersten und zweiten Seitenfläche eine Beschichtung auf. Insbesondere weist der Reinigungsartikel auf beiden Seitenflächen die erfindungsgemäße Beschichtung auf. Damit kann der Reinigungsartikel vom Anwender für intensivere Reinigungsvorgänge als auch einfacher, ohne Rücksicht auf die Orientierung des Reinigungsartikels, eingesetzt werden.

Der Reinigungsartikel ist vorzugsweise zur Reinigung der Haut, insbesondere Gesichtsreinigung, so als Kosmetikpad oder Kosmetiktuch, wie insbesondere als ein Peelingpad oder Peelingtuch einsetzbar. Neben kosmetischen Reinigungsartikeln ist der Reinigungsartikel auch zur Reinigung von harten Flächen einsetzbar. Insbesondere ist der Reinigungsartikel als Reinigungstuch, beispielsweise für Haushaltszwecke, insbesondere zur Geschirreinigung, aber auch zur Flächenreinigung, vorzugsweise im Küchen- oder Sanitärbereich, sowie zur Scheiben- und Fensterreinigung einsetzbar.

Bei dem Reinigungsartikel handelt es sich vorzugsweise um ein Einwegprodukt. Grundsätzlich sind jedoch auch Reinigungsartikel z.B. im Haushaltsbereich denkbar, die gewaschen oder gereinigt werden können.

Auf die vorliegende Weise kann ein Reinigungsartikel bereitgestellt werden, der besonders günstige Eigenschaften hinsichtlich Abrasiveigenschaft sowie Absorptionseigenschaft aufweist.

Weitere Merkmale und Einzelheiten sowie Vorteile der Erfindung ergeben sich aus der zeichnerischen Darstellung und nachfolgender Beschreibung des erfindungsgemäßen Reinigungsartikels. In der Zeichnung zeigen:
- Figur 1: eine Darstellung einer ersten Seitenfläche eines erfindungsgemäßen Reinigungsartikels,
- Figur 2: eine Darstellung einer ersten Seitenfläche eines weiteren erfindungsgemäßen Reinigungsartikels,
- Figur 3: eine Darstellung einer ersten Seitenfläche eines weiteren erfindungsgemäßen Reinigungsartikels,
- Figuren 4a - c): verschiedene Einzelmuster der Beschichtung,
- Figuren 5a - c): eine Darstellung der Bestimmung der Gitterquadrate und
- Figur 6: eine schematische, nicht maßstabsgetreue Darstellung eines Ausschnitts eines Shore-A Härtemessgeräts.

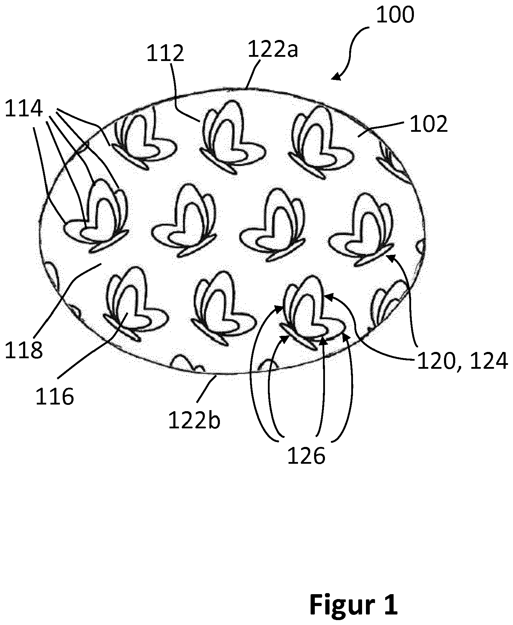

Figur 1 zeigt eine Draufsicht auf die erste Seitenfläche 102 eines erfindungsgemäßen Reinigungsartikels 100, in der Ausgestaltung eines Kosmetikpads, wobei die erste Seitenfläche 102 bei Anwendung zur Gesichtsreinigung der Haut des Benutzers zugewandt ist. Der Reinigungsartikel 100 besteht aus einem Grundmaterial aus Vliesmaterialien aus einer Mischung von natürlichen cellulosebasierten Fasern und synthetischen Fasern. Dieses Grundmaterial bildet eine Wattevliesschicht und ist dadurch verfestigt, dass durch partielles Schmelzen der synthetischen Fasern eine Verbindung der Fasermischung erhalten wird.
Im Falle von einem mehrlagigen Grundmaterial kann die Verbindung der Lagen über Kalandrieren, Kaschieren oder Laminieren erzielt werden.

Das Grundmaterial des Reinigungsartikels weist dabei eine Basislage mit einer Grammatur von vorzugsweise 80 - 300 g/m² auf.

Wie Figur 1 zeigt, ist auf der ersten Seitenfläche 102 des Reinigungsartikels 100 eine Beschichtung 112 aus Beschichtungslinien 114 vorgesehen. Die Beschichtung 112 dient dazu, Hautschuppen sowie Makeup etc. bei einer Gesichtsreinigung zu entfernen. Die Beschichtungslinien 114 sind polymerbasiert. Das Material der Beschichtung hat vorzugsweise eine Shore A-Härte von 70 - 95. Das Aufbringen der Beschichtungslinien erfolgt mittels eines Gravurverfahrens, wobei der Reinigungsartikel 100 zwischen einer Gravurwalze und einer Gegenwalze hindurchgeführt wird. Die Breite der Beschichtungslinien 114 beträgt im vorliegenden Fall 0,5 - 1,0 mm. Die Höhe der Beschichtungslinien beträgt vorzugsweise 0,2 - 0,4 mm, so dass durch das aufgebrachte Beschichtungsmuster keine unangenehmen haptischen Effekte auf der Haut auftreten.

Die in Figur 1 gezeigte Beschichtung weist eine Vielzahl von Einzelmustern 120 auf, die durch Beschichtungslinien 114 gebildet sind. Im dargestellten Fall ist bevorzugt jedes Einzelmuster 120 durch Mustergruppen 124 gebildet, wobei die Mustergruppen 124 hier aus vier Musterelementen 126 bestehen. Bei den vorliegenden Mustergruppen sind Musterelemente 126 vorhanden, die sowohl zueinander umlaufend als auch nebenaneinander angeordnet und dabei berührend sind. Zwischen den einzelnen Musterelementen jeder einzelnen ein Einzelmuster bildenden Mustergruppe ist keine Beschichtungsmasse aufgetragen, also darin liegt ein unbeschichteter Bereich 116 vor. Auf diese Weise wird durch die Beschichtungslinien 114 in Summe ein Bedeckungsgrad auf der ersten Seitenfläche von ca. 10-25% erreicht. Durch die Einzelelemente 120 als solche wird in Summe eine Flächenabdeckung von 30 - 40% der ersten Seitenfläche 102 erhalten, das heißt, die freien Flächen außerhalb der Einzelmuster 120, also die die Einzelmuster umgebende äußeren unbeschichteten Bereiche 118 nehmen ca. 60 -70% der ersten Seitenfläche 102 ein. Auf diese Weise kann die Abrasionskraft des Reinigungsartikels 100 auf der Haut in vorteilhafter Weise gestaltet werden bei gleichzeitig nur geringer Beeinträchtigung der dem Grundmaterial des Reinigungsartikels zugeschriebenen und erwünschten Eigenschaften, wie beispielsweise Weichheit und/oder Absorptionseigenschaften, die durch die Beschichtung nicht wesentlich beeinflusst werden.

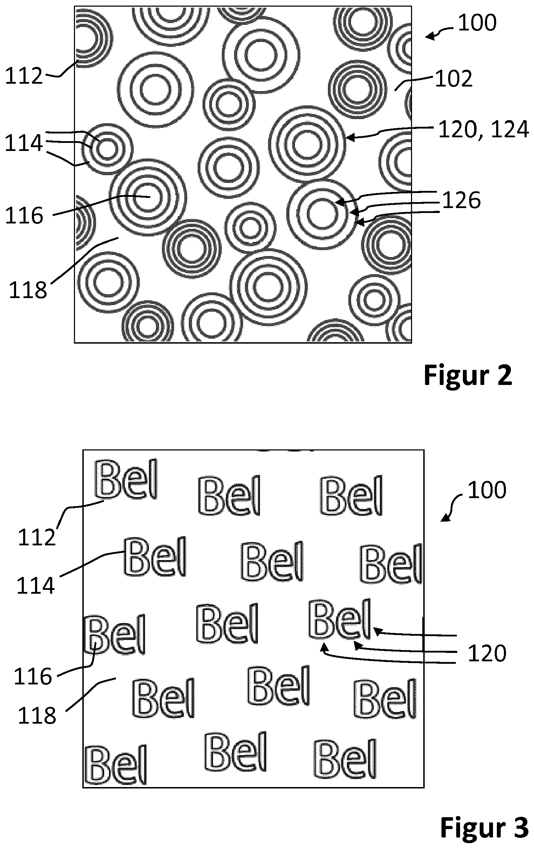

Figuren 2 und 3 zeigen weitere Ausführungsformen an Reinigungsartikel 100. So kann, wie in Figur 2 dargestellt, auf der ersten Seitenfläche 102 die Beschichtung 112 aus einer Vielzahl an aus Beschichtungslinien 114 aufgebauten Einzelmustern 120 bestehen, wobei die Einzelmuster 120 in Form von Mustergruppen 124 aus mindestens 3 Musterelementen 126, hier aus konzentrisch angeordneten Kreisen, ausgebildet sind. Damit ergeben sich innerhalb der Einzelmuster als auch außerhalb der Einzelmuster unbeschichtete Bereiche 116, 118. Die Einzelmuster können sich dabei auch schneiden, überlappen oder tangieren, wie aus Figur 2 ersichtlich ist.

Mit der Beschichtung 112 können auch aneinander gereihte, wortbildende, hier aus Beschichtungslinien 114 gebildete Einzelmuster 120 auf der ersten Seitenfläche des Reinigungsartikels erzeugt werden, wie in Figur 3 dargestellt ist. Auch hier ergeben sich innerhalb und außerhalb der Einzelmuster unbeschichtete Bereiche 116, 118.
Darüber hinaus bietet eine Beschichtung, bei der die Einzelmuster 120 sich zwar schneiden, überlappen oder tangieren können, jedoch jedes Einzelmuster für sich erkennbar bleibt und insbesondere nicht durch eine durchgehende Linie verbunden werden können, die von einer Seitenkante des Reinigungsartikels 122a zu einer gegenüberliegenden Seitenkante des Reinigungsartikels 122b verläuft, den Vorteil, dass keine Vorzugsrichtungen bestehen. Als Kanten der Reinigungsartikels 100 werden jeweils zwei gegenüberliegende Ränder des Reinigungsartikels 100 angesehen. Auf diese Weise können in sämtliche Richtungen die abrasiven Eigenschaften verbessert werden.

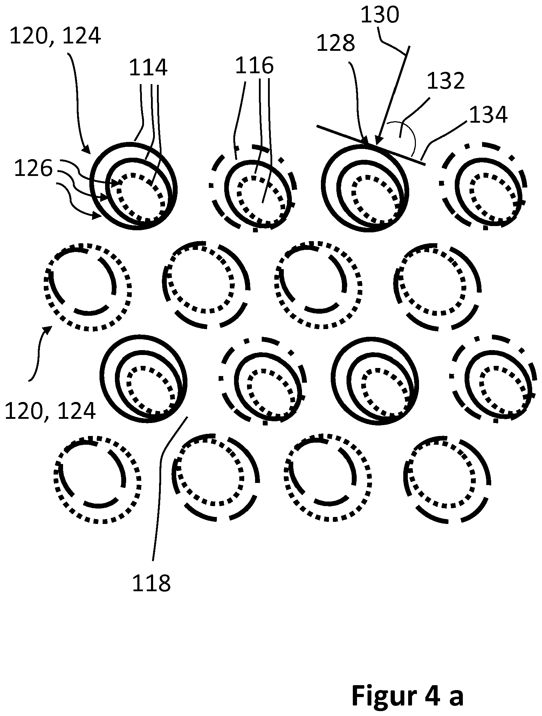

Besonders bevorzugt ist dabei eine Beschichtung, bei der aufgrund der Ausgestaltung der Einzelmuster 120 zumindest ein Einzelmuster 120, bevorzugt mindestens 20% der Einzelmuster 120 der ersten Seitenfläche, besonders bevorzugt jedes Einzelmuster 120, einen Abschnitt bzw. Bereich 128 aufweist, der senkrecht, also mit einem Winkel 132 von 90° zu einer beliebigen Richtung 130 in der Fläche des Reinigungsartikels 100 verläuft, so wie in Figur 4a schematisch dargestellt ist. Auf diese Weise kann jeder Bewegungsrichtung ein Anteil entgegengesetzt werden, der hierzu senkrecht verläuft und somit die optimale Abrasionswirkung für diese Bewegungsrichtung aufweist.

Die optimale Ausprägung der genannten Vorteile wird dadurch erreicht, dass die Einzelmuster 120 voneinander diskret sind und insbesondere nicht so ineinander übergehen, dass sich die Einzelmuster 120 in der Gesamtheit der Muster auflösen, wie es beispielsweise für die Einzelrauten oder Quadrate in einem Gittermuster der Fall ist.

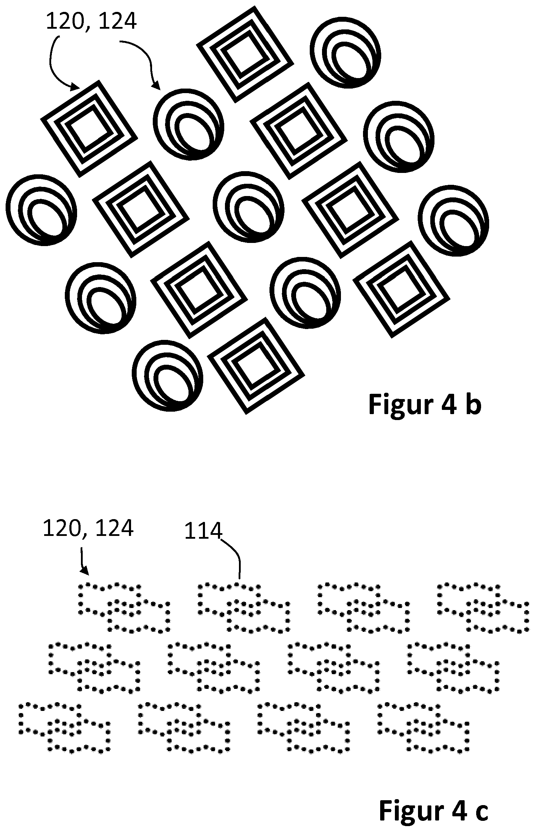

Weitere bevorzugte Einzelmuster zeigen die Figuren 4a - 4c, wobei sowohl verschiedene Einzelmuster miteinander kombiniert werden können, wie die Figuren 4a-4b zeigen, und darüber hinaus auch die Einzelmuster hinsichtlich der Ausbildung der Beschichtungslinien sowohl hinsichtlich deren Höhe als auch hinsichtlich deren Breite, einen Unterschied aufweisen können. Darüber hinaus ist es auch denkbar, die Beschichtungslinien nicht kontinuierlich durchgehend, sondern zumindest abschnittsweise unterbrochen auszuführen, wie gestrichelt, strichpunktiert oder gepunktet, wie es beispielsweise in Figur 4a gezeigt ist, sofern dies nicht zur Auflösung der Gesamtmuster derart führt, dass die Muster als solche nicht mehr erkannt werden können. Die aus Beschichtungslinien aufgebauten Einzelmuster können dabei auch vollständig aus Beschichtungslinien, die aus einer Kontinuität an Punkten zusammengesetzt sind, bestehen, wie beispielhaft in Figur 4c gezeigt ist.

Sofern sich ein Einzelmuster 120 als Mustergruppe 124 aus mehreren Musterelementen 126 zusammensetzt, können diese, wie in Figuren 4a und 4b dargestellt, einander voll umfänglich mit Abstand umlaufen, aber auch einander so umlaufen, dass Berührpunkte bestehen. Darüber hinaus ist es auch möglich, dass die einzelnen Musterelemente eines Einzelmusters 120 unter Bildung von Berühr- oder Schnittbereichen angeordnet sind, wie es beispielsweise Figur 4c zeigt. Auch die Einzelmuster gemäß den Figuren 4a bis 4c können analog zu Figur 2 so ausgebildet sein, dass die Einzelmuster einander schneiden, tangieren oder überlappen.

Der dynamische Reibungskoeffizient der beschichteten Seitenfläche des Reinigungsartikels nach Figur 1 beträgt gemessen in Anlehnung an ASTM D 1894-01 zwischen 0,4 und 0,7.

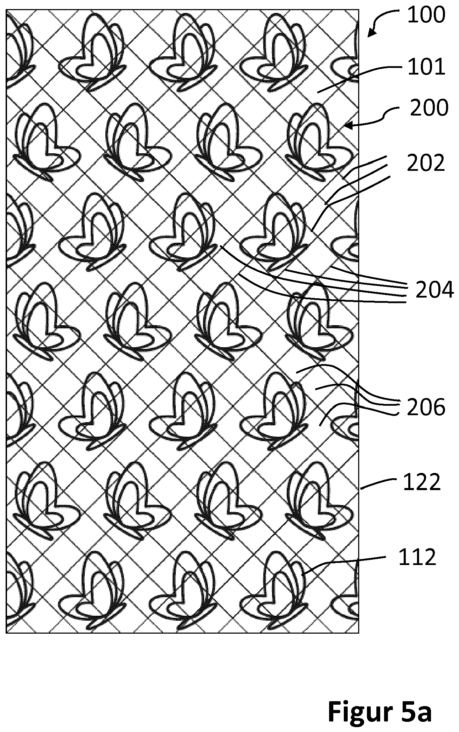

Zur Bemessung der Bedeckungsgrade auf der ersten Seitenfläche eines Reinigungsartikels wird ein imaginäres Gitternetz herangezogen. Die Bestimmung wird anhand der Figuren 5a-c näher erläutert:

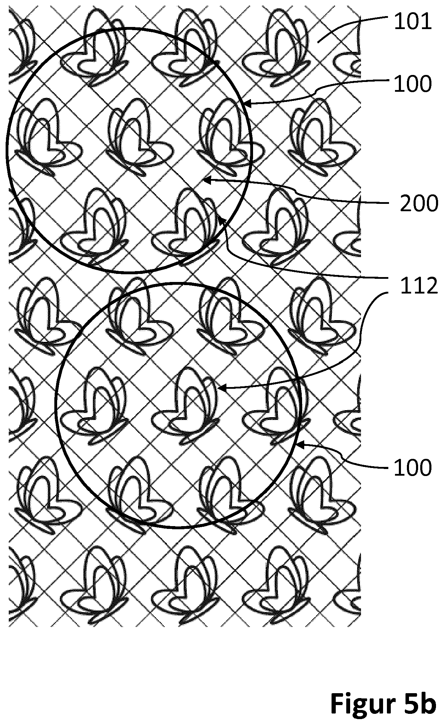

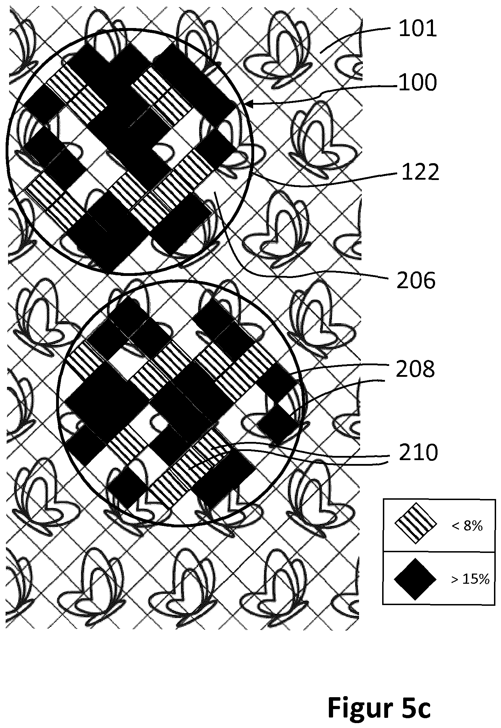

Figur 5a zeigt beispielhaft einen Reinigungsartikel 100 von rechteckiger Dimension mit der Aufsicht auf die erste Seitenfläche mit einer über die gesamte Erstreckung der ersten Seitenfläche verteilten Beschichtung 112. Ebenso soll anhand der Figur 5a beispielhaft ein schematischer Ausschnitt aus einem Flächenmaterial 101 mit einer darauf aufgebrachten Beschichtung 112 gezeigt werden. Aus dem Flächenmaterial 101 können beispielhaft dimensionsmäßig kleinere Reinigungsartikel 100, wie z.B. runde Kosmetikpads ausgestanzt oder ausgeschnitten werden, so wie in Figur 5b veranschaulicht ist. Zur Bemessung der Bedeckungsgrade wird ein imaginäres Gitternetz 200 herangezogen. Das Gitternetz 200, gebildet aus einer Anordnung an Linien, wobei eine erste Schar 202 aus parallelen, äquidistanten Linien durch eine zweite Schar 204 aus parallelen, äquidistanten Linien unter Bildung von rechten Winkeln geschnitten wird, besteht aus Gitterquadraten 206, jeweils mit einer Kantenlänge von 7 mm. Das Gitternetz kann in beliebiger Weise über einen Reinigungsartikel und dessen Beschichtung 112 aufgelegt werden. Zwar ist hier das Gitternetz 200 mit den Linienscharen in einem Winkel von 45° zur Kante 122 im Falle eines rechteckigen Reinigungsartikels vorgesehen, jedoch ist für die Bemessung keine Vorzugsrichtung zum Anlegen des Gitternetzes angedacht. Es werden die einzelnen Gitterquadrate 206 und der darin jeweils vorliegende Bedeckungsgrad ermittelt. Zur Auswertung werden Gitterquadrate pro 25 cm² mit einem ersten Bedeckungsgrad von mindestens 15% und Gitterquadrate pro 25 cm² mit einem zweiten Bedeckungsgrad von höchstens 8% zugrunde gelegt. Bei der Betrachtung von Reinigungsartikeln mit einer von 25 cm² abweichenden Dimension werden die auf der ersten Seitenfläche des Reinigungsartikels ermittelte Anzahl an Gitterquadraten mit einem ersten Bedeckungsgrad von mindestens 15% und die Anzahl an Gitterquadraten mit einem zweiten Bedeckungsgrad von höchstens 8% entsprechend auf 25 cm² normiert. Für die Auswertung des Bedeckungsgrades der Beschichtung in Bezug auf die jeweiligen Gitterquadrate können beliebige Auswerteverfahren, z.B. Computerunterstützte Grafik- und Zeichenprogramme herangezogen werden. Die Bemessung wird beispielhaft an Reinigungsartikeln 100 in der Ausgestaltung von runden Kosmetikpads, wie schematisch in Figur 5b dargestellt ist, gezeigt: Das Gitternetz 200 kann dabei beliebig aufgelegt sein und die Auswertung der Beschichtung 112 auf den Kosmetikpads 100 wird in Bezug auf die Gitterquadrate von 7 mm Kantenlange vorgenommen. Bei der Auswertung werden ausschließlich Gitterquadrate für die Ermittlung herangezogen, die vollständig im Bereich des Reinigungsartikels liegen. Gitterquadrate, die durch die Randlinie (Kante) 122 des Reinigungsartikels 100, also hier der Kosmetikpads, geschnitten werden, bleiben für die Betrachtung der zu zählenden Gitterquadrate außer Betracht. Bei den zwei Kosmetikpads aus Figur 5b weist die Beschichtung 21 bzw. 20 Gitterquadrate mit einem ersten Bedeckungsgrad von mindestens 15% und jeweils 10 Gitterquadrate mit einem zweiten Bedeckungsgrad von höchstens 8% auf, wie in Figur 5c weiter dargestellt ist. Die vollflächig ausgefüllten Gitterquadrate symbolisieren hierbei den ersten Bedeckungsgrad von mindestens 15% und die Gitterquadrate mit Streifenmuster symbolisieren den zweiten Bedeckungsgrad von höchstens 8%. Daraus ergibt sich für die Kosmetikpads eine Beschichtung mit einem Bedeckungsquotienten, ermittelt aus der Anzahl von Gitterquadraten des zweiten Bedeckungsgrades geteilt durch die Anzahl an Gitterquadraten des ersten Bedeckungsgrades, von 0,5. Diese Form an Auswertung kann an jeglichen Reinigungsartikeln unabhängig von deren geometrischen Form oder Dimension, wie bspw. rund, oval, eckig oder einer sonstigen Form, vorgenommen werden.

## Patentansprüche

1. Reinigungsartikel (100) mit einem absorbierenden Grundmaterial, das durch ein Flachmaterial (101) gebildet ist, mit einer ersten (102) und einer zweiten Seitenfläche, wobei auf mindestens der ersten Seitenfläche (102) eine Beschichtung (112) vorgesehen ist, die der ersten Seitenfläche (102) des Reinigungsartikels (100) eine gegenüber einer unbeschichteten ersten Seitenfläche (102) erhöhte Reibungskraft verleiht, **dadurch gekennzeichnet, dass** die Beschichtung (112) eine Vielzahl von Beschichtungslinien (114) umfasst und wobei, wenn über die erste Seitenfläche (102) des Reinigungsartikels (100) ein imaginäres Gitternetz (200) aus parallelen, äquidistanten, senkrecht zueinander verlaufenden Linien mit einer Kantenlänge von 7 mm eines jeden Gitterquadrats (206) gelegt wird, die erste Seitenfläche (102) im Bereich von mindestens 5 Gitterquadraten (208) pro 25 cm² einen ersten Bedeckungsgrad an Beschichtung von mindestens 15% und im Bereich von mindestens 5 Gitterquadraten (210) pro 25 cm² einen zweiten Bedeckungsgrad an Beschichtung von höchstens 8% aufweist.

2. Reinigungsartikel (100) nach Anspruch 1, **dadurch gekennzeichnet, dass** die erste Seitenfläche (102) im Bereich von mindestens 8, weiter insbesondere mindestens 10, weiter insbesondere mindestens 15, weiter insbesondere mindestens 20, weiter insbesondere höchstens 35, weiter insbesondere höchstens 30, weiter insbesondere höchstens 25 Gitterquadraten (208) pro 25 cm² einen ersten Bedeckungsgrad von mindestens 15% aufweist.

3. Reinigungsartikel (100) nach Anspruch 1 oder 2, **dadurch gekennzeichnet, dass** bei einer Beschichtung (112) mit mindestens 10, insbesondere mindestens 15 Gitterquadraten (208) pro 25 cm² mit dem ersten Bedeckungsgrad von mindestens 15 %, davon mindestens 5 Gitterquadrate einen Bedeckungsgrad von mindestens 20% aufweisen.

4. Reinigungsartikel (100) nach einem der vorangehenden Ansprüche, **dadurch gekennzeichnet, dass** die Gitterquadrate (208) pro 25cm² des ersten Bedeckungsgrades eine Obergrenze des Bedeckungsgrades von höchstens 50%, weiter insbesondere höchstens 40%, weiter insbesondere höchstens 30%, weiter insbesondere höchstens 25% aufweisen.

5. Reinigungsartikel (100) nach einem der vorangehenden Ansprüche, **dadurch gekennzeichnet, dass** die Beschichtung (112) einen Bedeckungsquotienten, ermittelt aus der Anzahl an Gitterquadraten (210) pro 25cm² des zweiten Bedeckungsgrades von höchstens 8% geteilt durch die Anzahl an Gitterquadraten (208) pro 25cm² des ersten Bedeckungsgrades von mindestens 15%, von mindestens 0,1, insbesondere mindestens 0,2, insbesondere mindestens 0,3, weiter insbesondere mindestens 0,4, weiter insbesondere höchstens 3,0, weiter insbesondere höchstens 2,5, weiter insbesondere höchstens 2,0, weiter insbesondere höchstens 1,8, weiter insbesondere höchstens 1,6 aufweist.

6. Reinigungsartikel (100) nach einem der vorangehenden Ansprüche, **dadurch gekennzeichnet, dass** die Beschichtung (112) mindestens einen Bereich, insbesondere mindestens 2 Bereiche , weiter insbesondere eine Vielzahl an Bereichen aufweist, in denen mindestens 2, insbesondere mindestens 3, insbesondere mindestens 4, weiter insbesondere höchstens 30, weiter insbesondere höchstens 25, weiter insbesondere höchstens 20 Gitterquadrate (208) des ersten Bedeckungsgrades von mindestens 15% direkt benachbart angeordnet sind und insbesondere mindestens einen Bereich, insbesondere mindestens 2 Bereiche, weiter insbesondere eine Vielzahl an Bereichen aufweist, in denen mindestens 2, insbesondere mindestens 3, weiter insbesondere mindestens 4 Gitterquadrate (210) des zweiten Bedeckungsgrades von höchstens 8% direkt benachbart angeordnet sind.

7. Reinigungsartikel (100) nach einem der vorangehenden Ansprüche, **dadurch gekennzeichnet, dass** die Beschichtung (112) eine Vielzahl von Einzelmustern (120), insbesondere aus Beschichtungslinien (114) gebildete Einzelmuster (120), aufweist oder daraus gebildet ist.

8. Reinigungsartikel (100) nach Anspruch 7, **dadurch gekennzeichnet, dass** mindestens ein Einzelmuster (120) mindestens einen Abschnitt (128) aufweist, der zu einer beliebigen Richtung in der ersten Seitenfläche (102) senkrecht verläuft, und insbesondere der mindestens eine Abschnitt (128) punktförmig ist und eine hieran angelegte imaginäre Tangente (134) senkrecht zu einer beliebigen Richtung (130) in der ersten Seitenfläche (102) verläuft.

9. Reinigungsartikel (100) nach einem der vorangehenden Ansprüche 7 bis 8, **dadurch gekennzeichnet, dass** zumindest ein Einzelmuster (120) als Mustergruppe (124) ausgebildet ist, die mindestens zwei aus Beschichtungslinien (114) gebildete Musterelemente (126) umfasst.

10. Reinigungsartikel (100) nach Anspruch 9, **dadurch gekennzeichnet, dass** ein erstes Musterelement (126) ein zweites Musterelement (126) oder weitere Musterelemente (126) zumindest bereichsweise, insbesondere vollständig umläuft und/oder dass ein erstes Musterelement (126) einem zweiten Musterelement (126) oder weiteren Musterelementen (126) nebeneinander zugeordnet und sich dabei berührend angeordnet ist.

11. Reinigungsartikel (100) nach einem der vorangehenden Ansprüche 7 - 10, **dadurch gekennzeichnet, dass** wenigstens ein Einzelmuster (120), insbesondere eine Vielzahl an Einzelmuster (120), insbesondere alle Einzelmuster (120) auf mindestens der ersten Seitenfläche (102) allseitig durch einen unbeschichteten äußeren Bereich umschlossen ist.

12. Reinigungsartikel (100) nach einem der vorangehenden Ansprüche, **dadurch gekennzeichnet, dass** die Beschichtung (112) die mindestens erste Seitenfläche (102) im Wesentlichen in ihrer gesamten Erstreckung erfasst.

13. Reinigungsartikel nach einem der vorangehenden Ansprüche, wobei die Beschichtung (112) gleichmäßig oder ungleichmäßig über der Reinigungsartikel (100) verteilt sein kann.

14. Reinigungsartikel (100) nach einem der vorangehenden Ansprüche, **dadurch gekennzeichnet, dass** die erste Seitenfläche (102) einen Bedeckungsgrad durch die Beschichtung (112) von mindestens 6%, insbesondere mindestens 8 %, weiter insbesondere mindestens 10%, weiter insbesondere mindestens 15%, weiter insbesondere mindestens 20 % und insbesondere von höchstens 50%, weiter insbesondere höchstens 40%, weiter insbesondere höchstens 30% und weiter insbesondere höchstens 25 % aufweist.

15. Reinigungsartikel (100) nach einem der vorangehenden Ansprüche, **dadurch gekennzeichnet, dass** die Beschichtungslinien (114) eine Linienbreite von mindestens 0,2 mm, insbesondere mindestens 0,4 mm, weiter insbesondere mindestens 0,5 mm, weiter insbesondere mindestens 0,6 mm, insbesondere von höchstens 2,0 mm, weiter insbesondere höchstens 1,6 mm, weiter insbesondere höchstens 1,2 mm, weiter insbesondere höchstens 1,0 mm aufweisen.

16. Reinigungsartikel (100) nach einem der vorangehenden Ansprüche, **dadurch gekennzeichnet, dass** die Beschichtungslinien (114) eine Länge aufweisen, die mindestens dem 5-fachen der Breite der jeweiligen Beschichtungslinie (114), vorzugsweise mindestens dem 6-fachen, weiter vorzugsweise mindestens dem 8-fachen und weiter vorzugsweise mindestens dem 10-fachen der Breite der jeweiligen Beschichtungslinie (114) entspricht.

17. Reinigungsartikel(100) nach einem der vorangehenden Ansprüche, **dadurch gekennzeichnet, dass** die Beschichtungslinien (114) eine Höhe von mindestens 0,1 mm, insbesondere mindestens 0,2 mm und insbesondere von höchstens 0,8 mm, weiter insbesondere von höchstens 0,6 mm und weiter insbesondere von höchstens 0,4 mm aufweisen.

18. Reinigungsartikel (100) nach einem der vorangehenden Ansprüche, **dadurch gekennzeichnet, dass** die Beschichtungslinien (114) durch durchgehende und/oder zumindest bereichsweise unterbrochene Linien, insbesondere gestrichelt, strichpunktiert, gepunktet, ausgebildet sind, wobei die Unterbrechung nicht länger als das 10-fache, insbesondere nicht länger als das 8-fache, insbesondere nicht länger als das 6-fache, insbesondere nicht länger als das 4-fache der Linienbreite der dieser unterbrochenen Stelle benachbarten Linie ist.

19. Reinigungsartikel (100) nach einem der vorangehenden Ansprüche, **dadurch gekennzeichnet, dass** die Beschichtung (112) ein Flächengewicht von mindestens 5 g/m², insbesondere mindestens 10 g/m², weiter insbesondere von mindestens 15 g/m², weiter insbesondere von mindestens 20 g/m², weiter insbesondere von höchstens 50 g/m², weiter insbesondere von höchstens 30 g/m² , weiter insbesondere auf höchstens 25 g/m² aufweist.

20. Reinigungsartikel (100) nach einem der vorangehenden Ansprüche, **dadurch gekennzeichnet, dass** die Beschichtung (112) polymerbasiert ist und aus Materialien mit einer Shore A-Härte von mindestens 30, insbesondere mindestens 40, weiter insbesondere mindestens 50, weiter insbesondere mindestens 60, insbesondere von höchstens 95, weiter insbesondere höchstens 90, weiter insbesondere höchstens 80, weiter insbesondere höchstens 70 gebildet ist.

21. Reinigungsartikel (100) nach einem der vorangehenden Ansprüche, **dadurch gekennzeichnet, dass** die erste Seitenfläche (102) mit der Beschichtung (112) einen dynamischen Reibungskoeffizienten in Anlehnung an ASTM D1894-01 von mindestens 0,2, insbesondere mindestens 0,3, weiter insbesondere mindestens 0,4, weiter insbesondere mindestens 0,5, weiter insbesondere mindestens 0,6, weiter insbesondere mindestens 0,7, weiter insbesondere von höchstens 1,5, insbesondere von höchstens 1,2, weiter insbesondere höchstens 1,0 aufweist.

## Claims

1. A cleaning article (100) having an absorbent base material formed by a flat material (101) having a first side face (102) and a second side face, wherein on at least the first side face (102)a coating (112) has been provided that imparts elevated abrasiveness to the first side face (102) of the cleaning article compared to an uncoated first side face (102), **characterized in that** the coating (112) comprises a multitude of coating lines (114) and wherein, when an imaginary grid (200) composed of parallel, equidistant lines running at right angles to one another with an edge length of 7 mm of each grid square (206) is placed over the first side face (102) of the cleaning article (100), the first side face (102) has a first degree of coverage of coating of at least 15% in the region of at least 5 grid squares (208) per 25 cm² and a second degree of coverage of coating of at most 8% in the region of at least 5 grid squares (210) per 25 cm².

2. The cleaning article (100) as claimed in claim 1, **characterized in that** the first side face (102) has a first degree of coverage of at least 15% in the region of at least 8, more particularly at least 10, more particularly at least 15, more particularly at least 20, more particularly at most 35, more particularly at most 30, more particularly at most 25 grid squares (208) per 25 cm².

3. The cleaning article (100) as claimed in claim 1 or 2, **characterized in that**, in the case of a coating (112) having at least 10, especially at least 15, grid squares (208) per 25 cm² having the first degree of coverage of at least 15%, at least 5 grid squares of these have a degree of coverage of at least 20%.

4. The cleaning article (100) as claimed in any of the preceding claims, **characterized in that** the grid squares (208) have an upper limit in the degree of coverage per 25 cm² of the first degree of coverage of at most 50%, more particularly at most 40%, more particularly at most 30%, more particularly at most 25%.

5. The cleaning article (100) as claimed in any of the preceding claims, **characterized in that** the coating (112) has a coverage quotient, ascertained from the number of grid squares (210) per 25 cm² of the second degree of coverage of not more than 8% divided by the number of grid squares (208) per 25 cm² of the first degree of coverage of at least 15%, of at least 0,1, particularly at least 0,2, particularly at least 0,3, more particularly at least 0,4, more particularly at most 3,0, more particularly at most 2,5, more particularly at most 2,0, more particularly at most 1,8, more particularly at most 1,6.

6. The cleaning article (100) as claimed in any of the preceding claims, **characterized in that** the coating (112) has at least one region, particularly at least 2 regions, more particularly a multitude of regions in which at least 2, particularly at least 3, particularly at least 4, more particularly at most 30, more particularly at most 25, more particularly at most 20, grid squares (208) of the first degree of coverage of at least 15% are in a directly adjacent arrangement, and particularly at least one region, particularly at least 2 regions, more particularly a multitude of regions in which at least 2, particularly at least 3, more particularly at least 4, grid squares (210) of the second degree of coverage of not more than 8% are in a directly adjacent arrangement.

7. The cleaning article (100) as claimed in any of the preceding claims, **characterized in that** the coating (112) has or has been formed from a multitude of individual patterns (120), especially individual patterns (120) formed from coating lines (114).

8. The cleaning article (100) as claimed in claim 7, **characterized in that** at least one individual pattern (120) has at least one section (128) that runs at right angles to any direction in the first side face (102), and in particular the at least one section (128) is in dot form and an imaginary tangent (134) applied thereto runs at right angles to any direction (130) in the first side face (102).

9. The cleaning article (100) as claimed in either of the preceding claims 7 and 8, **characterized in that** at least one individual pattern (120) takes the form of a group of patterns (124) comprising at least two pattern elements (126) formed from coating lines (114).

10. The cleaning article (100) as claimed in claim 9, **characterized in that** a first pattern element (126) encircles at least regions of and especially the entirety of a second pattern element (126) or further pattern elements (126), and/or **in that** a first pattern element (126) is assigned to and arranged in contact with a second pattern element (126) or further pattern elements (126) alongside.

11. The cleaning article (100) as claimed in any of the preceding claims 7-10, **characterized in that** at least one individual pattern (120), particularly a multitude of individual patterns (120), particularly all individual patterns (120), on at least the first side face (102) is surrounded on all sides by an uncoated outer region.

12. The cleaning article (100) as claimed in any of the preceding claims, **characterized in that** the coating (112) covers the at least first side face (102) essentially over its entire extent.

13. The cleaning article as claimed in any of the preceding claims, wherein the coating (112) may be distributed uniformly or non-uniformly across the cleaning article (100) .

14. The cleaning article (100) as claimed in any of the preceding claims, **characterized in that** the first side face (102) has a degree of coverage by the coating (112) of at least 6%, particularly at least 8%, more particularly at least 10%, more particularly at least 15%, more particularly at least 20%, and particularly at most 50%, more particularly at most 40%, more particularly at most 30%, and more particularly at most 25%.

15. The cleaning article (100) as claimed in any of the preceding claims, **characterized in that** the coating lines (114) have a line width of at least 0,2 mm, particularly at least 0,4 mm, more particularly at least 0,5 mm, more particularly at least 0,6 mm, particularly of at most 2,0 mm, more particularly at most 1,6 mm, more particularly at most 1,2 mm, more particularly at most 1,0 mm.

16. The cleaning article (100) as claimed in any of the preceding claims, **characterized in that** the coating lines (114) have a length corresponding to at least 5 times the width of the respective coating line (114), preferably at least 6 times, more preferably at least 8 times and more preferably at least 10 times the width of the respective coating line (114).

17. The cleaning article (100) as claimed in any of the preceding claims, **characterized in that** the coating lines (114) have a height of at least 0,1 mm, particularly at least 0,2 mm, and particularly of at most 0,8 mm, more particularly of at most 0,6 mm, more particularly of at most 0,4 mm.

18. The cleaning article (100) as claimed in any of the preceding claims, **characterized in that** the coating lines (114) take the form of continuous lines and/or lines that are interrupted at least in regions, especially in dashed, dashed-and-dotted or dotted form, where the interruption is not longer than 10 times, especially not longer than 8 times, especially not longer than 6 times, especially not longer than 4 times, the line width of the line adjacent to this site of interruption.

19. The cleaning article (100) as claimed in any of the preceding claims, **characterized in that** the coating (112) has a basis weight of at least 5 g/m², particularly at least 10 g/m², more particularly of at least 15 g/m², more particularly of at least 20 g/m², more particularly of at most 50 g/m², more particularly of at most 30 g/m², more particularly of at most 25 g/m².

20. The cleaning article (100) as claimed in any of the preceding claims, **characterized in that** the coating (112) is polymer-based and formed from materials having a Shore A hardness of at least 30, particularly at least 40, more particularly at least 50, more particularly at least 60, particularly of at most 95, more particularly at most 90, more particularly at most 80, more particularly at most 70.

21. The cleaning article (100) as claimed in any of the preceding claims, **characterized in that** the first side face (102) having the coating (112) has a dynamic coefficient of friction in accordance with ASTM D1894-01 of at least 0,2, particularly at least 0,3, more particularly at least 0,4, more particularly at least 0,5, more particularly at least 0,6, more particularly at least 0,7, more particularly of not more than 1,5, particularly of not more than 1,2, more particularly not more than 1,0.

## Revendications

1. Article de nettoyage (100) comprenant un matériau de base absorbant formé par un matériau plat (101), comprenant une première (102) et une deuxième surface latérale, sur au moins ladite première surface latérale (102) étant prévu un revêtement (112) qui confère à ladite première surface latérale (102) de l'article de nettoyage (100) une force de friction supérieure par rapport à une première surface latérale (102) non revêtue, **caractérisé par le fait que** le revêtement (112) présente une pluralité de lignes de revêtement (114), et dans lequel, lorsqu'un réseau de quadrillage imaginaire (200) constitué de lignes parallèles, équidistantes s'étendant perpendiculairement les unes aux autres, chaque carré (206) du quadrillage présentant une longueur d'arête de 7 mm, est appliqué sur la première surface latérale (102), ladite première surface latérale (102) présente, dans la zone d'au moins 5 carrés de quadrillage (208) par 25 cm², un premier degré de couverture de revêtement d'au moins 15 % et, dans la zone d'au moins 5 carrés de quadrillage (210) par 25 cm², un deuxième degré de couverture de revêtement de 8 % tout au plus.

2. Article de nettoyage (100) selon la revendication 1, **caractérisé par le fait que** la première surface latérale (102) présente un premier degré de couverture d'au moins 15 % dans la zone d'au moins 8, encore en particulier d'au moins 10, encore en particulier d'au moins 15, encore en particulier d'au moins 20, encore en particulier d'au plus 35, encore en particulier d'au plus 30, encore en particulier d'au plus 25 carrés de quadrillage (208) par 25 cm².

3. Article de nettoyage (100) selon la revendication 1 ou 2, **caractérisé par le fait que** dans un revêtement (112) comprenant au moins 10, en particulier au moins 15 carrés de quadrillage (208) par 25 cm² avec le premier degré de couverture d'au moins 15 %, au moins 5 carrés de quadrillage parmi ceux-ci présentent un degré de couverture d'au moins 20 %.

4. Article de nettoyage (100) selon l'une quelconque des revendications précédentes, **caractérisé par le fait que** les carrés de quadrillage (208) par 25 cm² du premier degré de couverture présentent une limite supérieure du degré de couverture de 50 % tout au plus, encore en particulier de 40 % tout au plus, encore en particulier de 30 % tout au plus, encore en particulier de 25 % tout au plus.

5. Article de nettoyage (100) selon l'une quelconque des revendications précédentes, **caractérisé par le fait que** le revêtement (112) présente un quotient de couverture - déterminé à partir du nombre de carrés de quadrillage (210) par 25 cm² du deuxième degré de couverture d'au plus 8 % divisé par le nombre de carrés de quadrillage (208) par 25 cm² du premier degré de couverture d'au moins 15 % - d'au moins 0,1, en particulier d'au moins 0,2, en particulier d'au moins 0,3, encore en particulier d'au moins 0,4, encore en particulier de 3,0 tout au plus, encore en particulier de 2,5 tout au plus, encore en particulier de 2,0 tout au plus, encore en particulier de 1,8 tout au plus, encore en particulier de 1,6 tout au plus.

6. Article de nettoyage (100) selon l'une quelconque des revendications précédentes, **caractérisé par le fait que** le revêtement (112) présente au moins une zone, en particulier au moins 2 zones, encore en particulier une pluralité de zones dans lesquelles au moins 2, en particulier au moins 3, en particulier au moins 4, encore en particulier 30 tout au plus, encore en particulier 25 tout au plus, encore en particulier au plus 20 carrés de quadrillage (208) du premier degré de couverture d'au moins 15 % sont disposés de manière à être directement adjacents, ainsi qu'en particulier au moins une zone, en particulier au moins 2 zones, encore en particulier une pluralité de zones dans lesquelles au moins 2, en particulier au moins 3, encore en particulier au moins 4 carrés de quadrillage (210) du deuxième degré de couverture de 8 % tout au plus sont disposés de manière à être directement adjacents.

7. Article de nettoyage (100) selon l'une quelconque des revendications précédentes, **caractérisé par le fait que** le revêtement (112) comprend une pluralité de motifs individuels (120), en particulier des motifs individuels (120) formés de lignes de revêtement (114), ou en est formé.

8. Article de nettoyage (100) selon la revendication 7, **caractérisé par le fait qu'**au moins un motif individuel (120) présente au moins une section (128) qui s'étend perpendiculairement à une direction quelconque dans la première surface latérale (102), et que, en particulier, ladite au moins une section (128) est en forme de point et une tangente imaginaire (134) appliquée à celle-ci s'étend perpendiculairement à une direction (130) quelconque dans la première surface latérale (102).

9. Article de nettoyage (100) selon l'une quelconque des revendications précédentes 7 à 8, **caractérisé par le fait qu'**au moins un motif individuel (120) est réalisé en tant que groupe de motifs (124) qui comprend au moins deux éléments de motif (126) formés à partir de lignes de revêtement (114).

10. Article de nettoyage (100) selon la revendication 9, **caractérisé par le fait qu'**un premier élément de motif (126) entoure au moins par zones, en particulier complètement, un deuxième élément de motif (126) ou d'autres éléments de motif (126) et/ou qu'un premier élément de motif (126) est associé à un deuxième élément de motif (126) ou à d'autres éléments de motif (126) tout en étant côte à côte et agencés en contact.

11. Article de nettoyage (100) selon l'une quelconque des revendications précédentes 7 à 10, **caractérisé par le fait qu'**au moins un motif individuel (120), en particulier une pluralité de motifs individuels (120), en particulier l'ensemble des motifs individuels (120) sur au moins la première surface latérale (102) sont entourés de tous côtés par une zone extérieure non revêtue.

12. Article de nettoyage (100) selon l'une quelconque des revendications précédentes, **caractérisé par le fait que** le revêtement (112) recouvre ladite au moins première surface latérale (102) pour l'essentiel dans toute son extension.

13. Article de nettoyage selon l'une quelconque des revendications précédentes, dans lequel le revêtement (112) peut être réparti uniformément ou non uniformément sur l'article de nettoyage (100).

14. Article de nettoyage (100) selon l'une quelconque des revendications précédentes, **caractérisé par le fait que** la première surface latérale (102) présente un degré de couverture par le revêtement (112) d'au moins 6 %, en particulier d'au moins 8 %, encore en particulier d'au moins 10 %, encore en particulier d'au moins 15 %, encore en particulier d'au moins 20 % et en particulier de 50 % tout au plus, encore en particulier de 40 % tout au plus, encore en particulier de 30 % tout au plus et encore en particulier de 25 % tout au plus.

15. Article de nettoyage (100) selon l'une quelconque des revendications précédentes, **caractérisé par le fait que** les lignes de revêtement (114) présentent une largeur de ligne d'au moins 0,2 mm, en particulier d'au moins 0,4 mm, encore en particulier d'au moins 0,5 mm, encore en particulier d'au moins 0,6 mm, en particulier de 2,0 mm tout au plus, encore en particulier de 1,6 mm tout au plus, encore en particulier de 1,2 mm tout au plus, encore en particulier de 1,0 mm tout au plus.

16. Article de nettoyage (100) selon l'une quelconque des revendications précédentes, **caractérisé par le fait que** les lignes de revêtement (114) présentent une longueur qui correspond au moins à 5 fois la largeur de la ligne de revêtement (114) respective, de préférence au moins à 6 fois, encore de préférence au moins à 8 fois et encore de préférence au moins à 10 fois la largeur de la ligne de revêtement (114) respective.

17. Article de nettoyage (100) selon l'une quelconque des revendications précédentes, **caractérisé par le fait que** les lignes de revêtement (114) présentent une hauteur de 0,1 mm au moins, en particulier de 0,2 mm au moins et en particulier de 0,8 mm tout au plus, encore en particulier de 0,6 mm tout au plus et encore en particulier de 0,4 mm tout au plus.

18. Article de nettoyage (100) selon l'une quelconque des revendications précédentes, **caractérisé par le fait que** les lignes de revêtement (114) sont formées par des lignes continues et/ou interrompues au moins par zones, en particulier en traits interrompus, en traits et points, en pointillés, l'interruption n'étant pas plus longue que 10 fois, en particulier n'étant pas plus longue que 8 fois, en particulier n'étant pas plus longue que 6 fois, en particulier n'étant pas plus longue que 4 fois la largeur de ligne de la ligne adjacente à ce point interrompu.

19. Article de nettoyage (100) selon l'une quelconque des revendications précédentes, **caractérisé par le fait que** le revêtement (112) présente un grammage d'au moins 5 g/m², en particulier d'au moins 10 g/m², encore en particulier d'au moins 15 g/m², encore en particulier d'au moins 20 g/m², encore en particulier de 50 g/m² tout au plus, encore en particulier de 30 g/m² tout au plus, encore en particulier de 25 g/m² tout au plus.

20. Article de nettoyage (100) selon l'une quelconque des revendications précédentes, **caractérisé par le fait que** le revêtement (112) est à base de polymère et est réalisé à partir de matériaux ayant une dureté Shore A d'au moins 30, en particulier d'au moins 40, encore en particulier d'au moins 50, encore en particulier d'au moins 60, en particulier de 95 tout au plus, encore en particulier de 90 tout au plus, encore en particulier de 80 tout au plus, encore en particulier de 70 tout au plus.

21. Article de nettoyage (100) selon l'une quelconque des revendications précédentes, **caractérisé par le fait que** la première surface latérale (102) avec le revêtement (112) présente un coefficient de frottement dynamique, suivant la norme ASTM D1894-01, d'au moins 0,2, en particulier d'au moins 0,3, encore en particulier d'au moins 0,4, encore en particulier d'au moins 0,5, encore en particulier d'au moins 0,6, encore en particulier d'au moins 0,7, encore en particulier de 1,5 tout au plus, en particulier de 1,2 tout au plus, encore en particulier de 1,0 tout au plus.
